(19) **Europäisches Patentamt**
**European Patent Office**
**Office européen des brevets**

(11) **EP 4 056 109 A1**

(12) # EUROPEAN PATENT APPLICATION
published in accordance with Art. 153(4) EPC

(43) Date of publication:
**14.09.2022 Bulletin 2022/37**

(51) International Patent Classification (IPC):
***A61B 5/026*** (2006.01)

(21) Application number: **19951826.7**

(86) International application number:
**PCT/CN2019/116664**

(22) Date of filing: **08.11.2019**

(87) International publication number:
**WO 2021/087967 (14.05.2021 Gazette 2021/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.11.2019 CN 201911066155**

(71) Applicant: **Suzhou Rainmed Medical Technology Co., Ltd.**
**Suzhou, Jiangsu 215000 (CN)**

(72) Inventors:
• **LIU, Guangzhi**
  **Suzhou, Jiangsu 215000 (CN)**
• **GONG, Yanjun**
  **Suzhou, Jiangsu 215000 (CN)**
• **LI, Jianping**
  **Suzhou, Jiangsu 215000 (CN)**
• **YI, Tieci**
  **Suzhou, Jiangsu 215000 (CN)**
• **ZHENG, Bo**
  **Suzhou, Jiangsu 215000 (CN)**

(74) Representative: **Boult Wade Tennant LLP**
**Salisbury Square House**
**8 Salisbury Square**
**London EC4Y 8AP (GB)**

(54) **METHOD AND APPARATUS FOR ACQUIRING BLOOD VESSEL ASSESSMENT PARAMETER ON BASIS OF PHYSIOLOGICAL PARAMETER, AND STORAGE MEDIUM**

(57) The present disclosure provides a method, an apparatus for acquiring blood vessel evaluation parameter based on physiological parameter, and a storage medium. The method for acquiring blood vessel evaluation parameter based on physiological parameter comprises acquiring a physiological parameter (S000); acquiring a blood flow velocity v (S020); acquiring, in real time, an aortic pressure waveform changing over time (S020); acquiring a coronary artery blood vessel evaluation parameter according to the blood flow velocity v, the aortic pressure waveform and the physiological parameter (S030). The coronary artery blood vessel evaluation parameter is acquired according to the blood flow velocity v, the aortic pressure waveform and the physiological parameter. And this individualized measurement of coronary artery blood vessel evaluation parameters for patients with different genders and differential disease histories is more targeted and improves the accuracy of coronary artery blood vessel evaluation parameters.

FIG.1

EP 4 056 109 A1

**Description**

TECHNICAL FIELD

The present disclosure relates to the field of coronary artery technology, and in particular, to a method and an apparatus for acquiring coronary artery blood vessel evaluation parameter based on physiological parameter, a coronary artery analysis system and a computer storage medium.

BACKGROUND

According to the statistics of the World Health Organization, cardiovascular diseases have become a "leading killer" of human health. In recent years, the analysis of the physiological and pathological behaviors of cardiovascular diseases using hemodynamics has also become a very important means of diagnosis of the cardiovascular diseases.

Blood flow quantity and flow velocity are very important parameters of hemodynamics. How to measure the blood flow quantity and flow velocity accurately and conveniently has become the focus of many researchers.

A coronary artery blood vessel evaluation parameter comprises a coronary artery blood flow velocity CAIFR during a diastolic phase, and an index for microcirculatory resistance of coronary artery CAIFMR during the diastolic phase, etc.; however, due to different vital signs of different populations, evaluation standards for normal values are slightly different. For example, the myocardial microcirculation function of the elderly is relatively poor, and the blood flow velocity is generally lower than that of the young. If the industry general evaluation standard is used, the blood flow velocity used will be higher than the actual value, leading to underestimation of CAIFMR and the like, and reducing the measurement accuracy of CAIFMR and the like.

SUMMARY

The present disclosure provides a method and an apparatus for acquiring coronary artery blood vessel evaluation parameter based on physiological parameter, a coronary artery analysis system and a computer storage medium, so as to solve the problem of inaccurate measurement of coronary artery blood vessel evaluation parameter caused by slightly different evaluation standards for normal values due to different populations having different vital signs.

In order to achieve the above object, in a first aspect, the present disclosure provides a method for acquiring coronary artery blood vessel evaluation parameter based on physiological parameter comprising:

acquiring a physiological parameter;
acquiring a blood flow velocity v;

acquiring, in real time, an aortic pressure waveform changing over time;
acquiring a coronary artery blood vessel evaluation parameter according to the blood flow velocity v, the aortic pressure waveform and the physiological parameter.

Optionally, in the above method for acquiring coronary artery blood vessel evaluation parameter based on physiological parameter, the coronary artery blood vessel evaluation parameter is an index for microcirculatory resistance of coronary artery CAIFMR during a diastolic phase, comprising:

selecting a maximum value of the blood flow velocity v, as a maximum blood flow velocity $v_{max}$ during the diastolic phase;
as a time period corresponding to the $v_{max}$ being the diastolic phase, acquiring an average aortic pressure during the diastolic phase according to the aortic pressure waveform;

$$\mathrm{CAIFMR} = \overline{P_a} \big/ v_{max} \times k + c \,;$$

$$\overline{P_a} = \frac{\sum_1^j \big(P_{a1} + P_{a2} \ldots P_{aj}\big)}{j} \,;$$

wherein, $\overline{P_a}$ represents the average aortic pressure during the diastolic phase; $P_{a1}$, $P_{a2}$, and $P_{aj}$ respectively represent the aortic pressures corresponding to a first point, a second point, and a *j-th* point within the diastolic phase on the aortic pressure waveform, and *j* represents the number of pressure points contained in the aortic pressure waveform during the diastolic phase, $v_{max}$ represents the maximum blood flow velocity during the diastolic phase obtained by selecting the maximum value from all blood flow velocities v; k=axb, a represents a characteristic value of diabetes, and b represents a characteristic value of hypertension, and c represents gender.

Optionally, in the above method for acquiring coronary artery blood vessel evaluation parameter based on physiological parameter, if a patient does not suffer from diabetes, then $0.5 \le a \le 1$; if the patient suffers from diabetes, then $1 < a \le 2$;

if the patient's blood pressure is greater than or equal to 90 mmHg, then $1 < b \le 1.5$; if the patient's blood pressure is less than 90 mmHg, then $0.5 \le b \le 1$;
if the patient is male, then c=0; if the patient is female, then c=3~10.

Optionally, in the above method for acquiring coronary artery blood vessel evaluation parameter based on physiological parameter, if the patient does not suffer from diabetes, then a=1; if the patient suffers from diabetes, then a=2;

if the patient's blood pressure is greater than or equal to 90mmHg, then the=1.5; if a patient's blood pressure is less than 90mmHg, then b=1;
if the patient is male, c=0; if the patient is female, c=5.

Optionally, in the above method for acquiring coronary artery blood vessel evaluation parameter based on physiological parameter, a manner for acquiring a blood flow velocity comprises:

reading a group of two-dimensional coronary artery angiogram images of at least one body position;
extracting a blood vessel segment of interest from the group of two-dimensional coronary artery angiogram images;
extracting a centerline of the blood vessel segment;
determining a difference in time taken for a contrast agent flowing through the blood vessel segment in any two frames of the two-dimensional coronary artery angiogram images with the difference being $\Delta t$, and determining a difference for the centerline of a sub-segment of the blood vessel segment in the two frames of two-dimensional coronary artery angiogram image with the difference being $\Delta L$;
solving the blood flow velocity according to the ratio of $\Delta L$ to $\Delta t$.

Optionally, in the above method for acquiring coronary artery blood vessel evaluation parameter based on physiological parameter, a manner for reading a group of two-dimensional coronary artery angiogram images of at least one body position comprises:

directly reading the group of two-dimensional coronary artery angiogram images of at least one body position from an angiogram image capturing device or a hospital platform in a wireless or wired way; or reading the group of two-dimensional coronary artery angiogram images of at least one body position via a storage device.

Optionally, in the above method for acquiring coronary artery blood vessel evaluation parameter based on physiological parameter, a manner for extracting a blood vessel segment of interest from the group of two-dimensional coronary artery angiogram images comprises:

selecting N frames of the two-dimensional coronary artery angiogram images from the group of two-dimensional coronary artery angiogram images;
acquiring the blood vessel segment of interest by picking a beginning point and an ending point of the blood vessel of interest on the two-dimensional coronary artery angiogram images.

Optionally, in the above method for acquiring coronary artery blood vessel evaluation parameter based on physiological parameter, a manner for extracting the centerline of the blood vessel segment comprises:

extracting a blood vessel skeleton from the two-dimensional coronary artery angiogram images;
according to the extension direction of the blood vessel segment and the principle of obtaining the shortest path between two points;
extracting the centerline of the blood vessel segment along the blood vessel skeleton.

Optionally, in the above method for acquiring coronary artery blood vessel evaluation parameter based on physiological parameter, a manner for extracting the centerline of the blood vessel segment along the blood vessel skeleton comprises:

adding at least one seed point on the blood vessel segment of interest;
regenerating the centerline of the blood vessel along the blood vessel skeleton according to the beginning and ending points and the seed point.

Optionally, in the above method for acquiring coronary artery blood vessel evaluation parameter based on physiological parameter, a manner for determining a difference in time taken for a contrast agent flowing through the blood vessel segment in any two frames of the two-dimensional coronary artery angiogram images with the difference being $\Delta t$, and determining a difference for the centerline of a sub-segment of the blood vessel segment in the two frames of two-dimensional coronary artery angiogram image with the difference being $\Delta L$, and solving the blood flow velocity according to the ratio of $\Delta L$ to $\Delta t$ comprises:

taking the coronary angiogram image when the contrast agent flows to the inlet of the coronary artery, that is, the beginning point of the blood vessel segment as a first frame of image, and taking the coronary angiogram image when the contrast agent flows to the ending point of the blood vessel segment as a N-th frame of image;
solving the time difference and centerline length difference of the N-th frame of image and a (N-1)th frame, ..., a (N-b)th frame, ..., a (N-a)th frame, ..., the first frame of image, successively, with the time differences being $\Delta t_1$,..., $\Delta t_b$,..., $\Delta t_a$,..., $\Delta t_{N-1}$, respectively; the centerline length differences being $\Delta L_1$,..., $\Delta L_b$,..., $\Delta L_a$,..., $\Delta L_{N-1}$, respectively;

according to $v = \dfrac{\Delta L}{\Delta t}$, obtaining the blood flow velocity from the N-th frame of image to the (N-1)th frame, ..., the (N-b)th frame, ..., the (N-a)th frame, ..., the first frame of image, respectively, wherein v represents the blood flow velocity, with the blood flow velocity being $v_1$,..., $v_b$,..., $v_a$,..., $v_{N-1}$, respectively.

Optionally, in the above method for acquiring coronary artery blood vessel evaluation parameter based on physiological parameter, a manner for determining a difference in time taken a contrast agent flowing through the blood vessel segment in any two frames of the two-dimensional coronary artery angiogram images with the difference being $\Delta t$, and determining a difference for the centerline of a sub-segment of the blood vessel segment in the two frames of two-dimensional coronary artery angiogram image with the difference being $\Delta L$, and solving the blood flow velocity according to the ratio of $\Delta L$ to $\Delta t$ comprises:

solving the time difference and centerline length difference of the N-th frame and b-th frame, of the (N-1)th frame and (b-1)th frame, ..., of the (N-b-a)th frame and (N-a)th frame, ..., of the (N-b+1)th frame and first frame of image, successively;

according to $v = \dfrac{\Delta L}{\Delta t}$, obtaining the blood flow velocity from the N-th frame to the b-th frame, from the (N-1)th frame to the (b-1)th frame, ..., from the (N-b-a)th frame to the (N-a)th frame, ..., from the (N-b+1)th frame to the first frame of image, respectively, wherein v represents the blood flow velocity.

Optionally, in the above method for acquiring coronary artery blood vessel evaluation parameter based on physiological parameter, a manner for selecting a maximum value of the blood flow velocity as a blood flow velocity during the diastolic phase comprises:

selecting a maximum value of the blood flow velocity from claim 11 or claim 12 as a blood flow velocity during the diastolic phase through a recursive algorithm or a bubbling algorithm; or
selecting a maximum value of the blood flow velocity from claim 11 or claim 12 as a blood flow velocity during the diastolic phase through a recursive algorithm or a bubbling algorithm; selecting a minimum value the blood flow velocity as a blood flow velocity during a systolic phase.

Optionally, in the above method for acquiring coronary artery blood vessel evaluation parameter based on physiological parameter, after the manner for extracting a centerline of the blood vessel segment, and before the manner for determining a difference in time taken for a contrast agent flowing through the blood vessel segment in any two frames of the two-dimensional coronary artery angiogram images with the difference being $\Delta t$, and determining a difference for the centerline of a sub-segment of the blood vessel segment in the two frames of two-dimensional coronary artery angiogram image with the difference being $\Delta L$, the method further comprises:

reading a group of two-dimensional coronary artery angiogram images of at least two body positions;
acquiring geometric structure information of the blood vessel segment, comprising the physiological parameter, and image shooting angles of the body positions;
performing graphics processing on the blood vessel segment of interest;
extracting a blood vessel contour line of the blood vessel segment;
according to the geometric structure information of the blood vessel segment, synthesizing a three-dimensional blood vessel model by projecting the two-dimensional coronary angiogram images of the at least two body positions with extracted centerline and contour line of the blood vessel onto a three-dimensional plane.

Optionally, in the above method for acquiring coronary artery blood vessel evaluation parameter based on physiological parameter, a manner for solving the blood flow velocity according to the ratio of $\Delta L$ to $\Delta t$ comprises:

according to the three-dimensional blood vessel model, acquiring a centerline of the three-dimensional blood vessel model, correcting the centerline extracted by the two-dimensional coronary angiogram images, and correcting the centerline difference $\Delta L$ to obtain $\Delta L'$;
solving the blood flow velocity v according to the ratio of the $\Delta L'$ to the $\Delta t$.

In a second aspect, the present disclosure provides an apparatus for acquiring coronary artery blood vessel evaluation parameter based on physiological parameter, for use in the method for acquiring coronary artery blood vessel evaluation parameter based on physiological parameter according to any one of the above, comprising: a blood flow velocity acquisition unit, an aortic pressure waveform acquisition unit, and a coronary artery blood vessel evaluation parameter unit, the coronary artery blood vessel evaluation parameter unit being connected to the blood flow velocity acquisition unit and the aortic pressure waveform acquisition unit.

The blood flow velocity obtaining unit is configured to obtain a blood flow velocity v;

The aortic pressure waveform acquisition unit is configured to acquire, in real time, an aortic pressure waveform changing over time;

The coronary artery blood vessel evaluation parameter unit is configured to receive the blood flow velocity v and the aortic pressure waveform sent by the blood flow velocity acquisition unit and the aortic pressure waveform acquisition unit, and then to obtain a coronary artery evaluation parameter according to a physiological parameter.

Optionally, the above apparatus for acquiring coronary artery blood vessel evaluation parameter based on physiological parameter, further comprises: an image reading unit, a blood vessel segment extraction unit, and a centerline extraction unit connected in sequence, a time difference unit and a geometric information acquisition unit connected to the image reading unit. The blood flow velocity acquisition unit is connected to the time difference unit and the centerline difference unit, respectively. The centerline difference unit is connected with the centerline extraction unit. The geometric information acquisition unit is connected with the coronary artery blood vessel evaluation parameter unit.

The image reading unit is configured to read a group of two-dimensional coronary artery angiogram image of at least one body position;

The blood vessel segment extraction unit is configured to receive two-dimensional coronary artery angiogram images sent by the image reading unit, and to extract a blood vessel segment of interest in the images.

The centerline extraction unit is configured to receive the blood vessel segment sent by the blood vessel segment extraction unit, and to extract the centerline of the blood vessel segment.

The time difference unit is configured to receive any two frames of the two-dimensional coronary artery angiogram images sent by the image reading unit, and to determine a difference in time taken for a contrast agent flowing through the blood vessel segment in the two frames of two-dimensional coronary artery angiogram image with the difference being $\varDelta t$.

The centerline difference unit is configured to receive the centerline of a sub-segment of the blood vessel segment flowed through by the contrast agent in the two frames of two-dimensional coronary artery angiogram image sent by the centerline extraction unit, and to determine a difference for the centerline of a sub-segment of the blood vessel segment in the two frames of two-dimensional coronary artery angiogram image with the difference being $\varDelta L$.

The blood flow velocity acquisition unit comprises a blood flow velocity calculation module and a diastolic blood flow velocity calculation module, the blood flow velocity calculation module being respectively connected to the time difference unit and the centerline difference unit, the diastolic blood flow velocity calculation module being connected with the blood flow velocity calculation module.

The blood flow velocity calculation module is configured to receive the $\varDelta L$ and the $\varDelta t$ sent by the time difference unit and the centerline difference unit, and to solve the blood flow velocity according to the ratio of $\varDelta L$ to $\varDelta t$.

The diastolic blood flow velocity calculation module is configured to receive the blood flow velocity sent by the blood flow velocity calculation module, and to select a maximum value of the blood flow velocity as a blood flow velocity during a diastolic phase;

The geometric information acquisition unit is configured to receive the two-dimensional coronary artery angiogram images of the image reading unit, to acquire a physiological parameter of a patient and image shooting angles, and to transmit the physiological parameter and image shooting angles to the coronary artery blood vessel evaluation parameter unit.

Optionally, the above apparatus for acquiring coronary artery blood vessel evaluation parameter based on physiological parameter further comprises: a blood vessel skeleton extraction unit and a three-dimensional blood vessel reconstruction unit, both connected to the image reading unit, a contour line extraction unit connected to the blood vessel skeleton extraction unit, the three-dimensional blood vessel reconstruction unit being connected with the geometric information acquisition unit, the centerline extraction unit and the contour line extraction unit.

The blood vessel skeleton extraction unit is configured to receive the two-dimensional coronary artery angiogram images sent by the image reading unit, and to extract a blood vessel skeleton in the images.

The contour line extraction unit is configured to receive the blood vessel skeleton of the blood vessel skeleton extraction unit, and to extract a contour line of the blood vessel segment of interest according to the blood vessel skeleton.

The three-dimensional blood vessel reconstruction unit is configured to receive the contour line, the image shooting angles and the centerline sent by the contour line extraction unit, the geometric information acquisition unit and the centerline extraction unit, and to receive the two-dimensional coronary artery angiogram images sent by the image reading unit in order to synthesize a three-dimensional blood vessel model by projecting the two-dimensional coronary angiogram images of at least two body positions with extracted centerline and contour line of the blood vessel onto a three-dimensional plane according to the geometric structure information of the blood vessel segment.

The centerline extraction unit is configured to re-extract the centerline of the blood vessel segment from the three-dimensional blood vessel model of the three-dimensional blood vessel reconstruction unit, and to re-acquire the length of the centerline.

In a third aspect, the present disclosure provides a coronary artery analysis system comprising the apparatus for acquiring coronary artery blood vessel evaluation parameter based on physiological parameter according to any one of the above.

In a fourth aspect, the present disclosure provides a computer storage medium, wherein the method for acquiring coronary artery blood vessel evaluation parameter based on physiological parameter according to any one of the above is implemented when a computer program is executed by a processor.

The beneficial effects brought about by the solutions provided by the embodiments of the present disclosure comprise at least the following.

The present disclosure provides a method for acquiring coronary artery blood vessel evaluation parameter based on physiological parameter, wherein a coronary vascular evaluation parameter is obtained according to a blood flow velocity v, an aortic pressure waveform, and a physiological parameter. And this individualized measurement of coronary artery blood vessel evaluation parameters for patients with different genders and differential disease histories is more targeted and improves the accuracy of coronary artery blood vessel evaluation parameters.

BRIEF DESCRIPTION OF THE DRAWINGS

The drawings illustrated here are used to provide a further understanding of the present disclosure and constitute a part of the present disclosure. The exemplary embodiments and the descriptions thereof are used to explain the present disclosure, and do not constitute an improper limitation on the present disclosure. In the drawings:

    FIG. 1 is a flowchart of a method for acquiring coronary artery blood vessel evaluation parameter based on physiological parameter of the present disclosure;

    FIG. 2 is a flowchart of acquiring a blood flow velocity by a two-dimensional angiogram image in S010 of the present disclosure;

    FIG. 3 is a flowchart of acquiring a blood flow velocity by three-dimensional modeling in S010 of the present disclosure;

    FIG. 4 is a flowchart of S200 of the present disclosure;

    FIG. 5 is a flow chart of S300 of the present disclosure;

    FIG. 6 is a flowchart of S330 of the present disclosure;

    FIG. 7 is a flowchart of the method (1) of S400A or S700B of the present disclosure;

    FIG. 8 is a flowchart of the method (2) of S400A or S700B of the present disclosure;

    FIG. 9 is a structural block diagram of an embodiment of an apparatus for acquiring coronary artery blood vessel evaluation parameter based on physiological parameter of the present disclosure;

    FIG. 10 is a structural block diagram of another embodiment of an apparatus for acquiring coronary artery blood vessel evaluation parameter based on physiological parameter according to the present disclosure;

Reference numerals are explained below:

blood flow velocity acquisition unit 1, blood flow velocity calculation module 101, diastolic blood flow velocity calculation module 102, aortic pressure waveform acquisition unit 2, coronary artery blood vessel evaluation parameter unit 3, image reading unit 4, blood vessel segment extraction unit 5, centerline extraction unit 6, time difference unit 7, geometric information acquisition unit 8, centerline difference unit 9, blood vessel skeleton extraction unit 10, three-dimensional blood vessel reconstruction unit 11, and contour line extraction unit 12.

DETAILED DESCRIPTION OF PREFERRED EMBODIMENTS

In order to make objects, technical solutions and advantages of the present disclosure clearer, the technical solutions of the present disclosure will be clearly and completely described below with reference to the specific embodiments and corresponding drawings. It is apparent that the described embodiments are merely part of the embodiments of the present disclosure rather than all of them. Based on the embodiments in the present disclosure, without making creative work, all the other embodiments obtained by a person skilled in the art will fall into the protection scope of the present disclosure.

Hereinafter, a number of embodiments of the present disclosure will be disclosed with drawings. For clear illustration, many practical details will be described in the following description. However, it should be understood that the present disclosure should not be limited by these practical details. In other words, in some embodiments of the present disclosure, these practical details are unnecessary. In addition, in order to simplify the drawings, some conventionally used structures and components will be shown in simple schematic ways in the drawings.

A coronary artery blood vessel evaluation parameter comprises a coronary artery blood flow velocity CAIFR during a diastolic phase, an index for microcirculatory resistance of coronary artery CAIFMR during the diastolic phase, etc.; however, due to different vital signs of different populations, evaluation standards for normal values are slightly different. For example, the myocardial microcirculation function of the elderly is relatively poor, and the blood flow velocity is generally lower than that of the young. If the industry general evaluation standard is used, the blood flow velocity used will be higher than the actual value, leading to underestimation of CAIFMR and the like, and reducing the measurement accuracy of CAIFMR and the like.

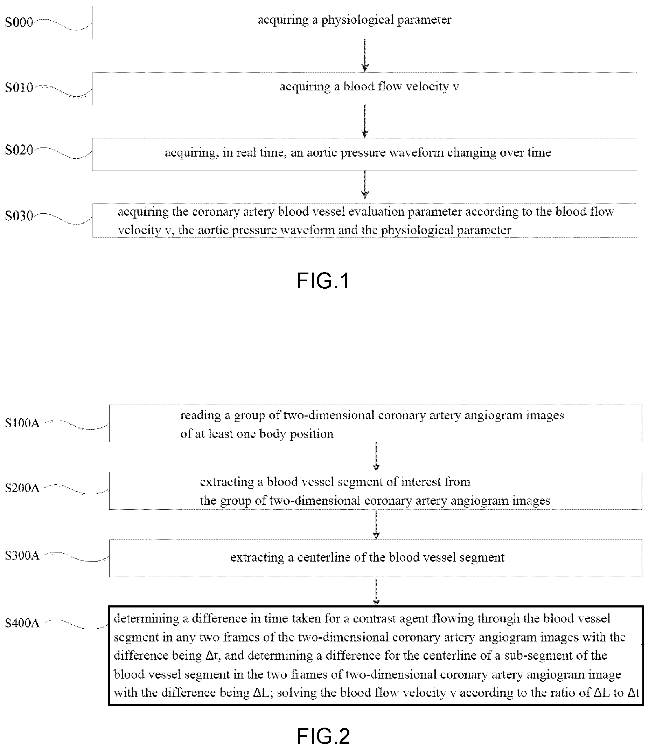

In order to solve the above problem, as shown in FIG. 1, the present disclosure provides a method for acquiring coronary artery blood vessel evaluation parameter based on physiological parameter, comprising:

S000, acquiring a physiological parameter;
S010, acquiring a blood flow velocity v;
S020, acquiring, in real time, an aortic pressure waveform changing over time;
S030, acquiring the coronary artery blood vessel evaluation parameter according to the blood flow velocity v, the aortic pressure waveform and the physiological parameter;
The present disclosure provides a method for acquiring coronary artery blood vessel evaluation parameter based on physiological parameter, wherein a coronary vascular evaluation parameter is obtained according to a blood flow velocity v, an aortic pressure waveform, and a physiological parameter. And this individualized measurement of coronary artery blood vessel evaluation parameters for patients with different genders and differential disease histories is more targeted and improves the accuracy of coronary artery blood vessel evaluation parameters.

All the contents of acquiring coronary artery blood vessel evaluation parameter based on the physiological parameter are within the scope of protection of the present disclosure. The following is a description of the specific acquisition manner for the case that the coronary artery evaluation parameter is an index for microcirculatory resistance of coronary artery CAIFMR during the diastolic phase.

Embodiment 1: after a large number of experimental verifications, histories of having hypertension and diabetes and gender all have impacts on the calculation accuracy of the coronary artery blood vessel evaluation parameter, and thus, for the case that the coronary artery evaluation parameter in S030 is the index for microcirculatory resistance CAIFMR during the diastolic phase, the specific steps comprise:

selecting a maximum value of the blood flow velocity v, as a maximum blood flow velocity $v_{max}$ during the diastolic phase; preferably, in the present disclosure, selecting the maximum value of the blood flow velocity v through a recursive algorithm or a bubbling algorithm;
the period corresponding to $v_{max}$ is the diastolic phase, and an average aortic pressure during the diastolic phase is acquired according to the aortic pressure waveform;

$$CAIFMR = \overline{P_a}/v_{max} \times k + c \; ;$$

$$\overline{P_a} = \frac{\sum_1^j \left( P_{a1} + P_{a2} \ldots P_{aj} \right)}{j} \; ;$$

wherein, $\overline{P_a}$ represents the average aortic pressure during the diastolic phase; $P_{a1}$, $P_{a2}$, and $P_{aj}$ represent aortic pressures corresponding to a first point, a second point, and a j-th point within the diastolic phase on the aortic pressure waveform, respectively, and j represents the number of pressure points contained in the aortic pressure waveform during the diastolic phase, $v_{max}$ represents the maximum blood flow velocity during the diastolic phase, which is obtained by selecting the maximum value from all blood flow velocities v; $k=a \times b$, a represents a characteristic value of diabetes, and b represents a characteristic value of hypertension, and c represents gender.

In an embodiment of the present disclosure, if a patient does not suffer from diabetes, then $0.5 \leq a \leq 1$, preferably, a=1; if the patient suffers from diabetes, then $1 < a \leq 2$, preferably, a=2;

if the patient's blood pressure is greater than or equal to 90 mmHg, then $1 < b \leq 1.5$, preferably, b=1.5; if the patient's blood pressure is less than 90 mmHg, then $0.5 \leq b \leq 1$, preferably, b=1;
if the patient is male, then c=0; if the patient is female, then c=3~10, preferably, c=5.

As shown in FIG. 2 , in an embodiment of the present disclosure, when the blood flow velocity is acquired through a two-dimensional angiogram image, S010 comprises:

S100A, reading a group of two-dimensional coronary artery angiogram images of at least one body position;
S200A, extracting a blood vessel segment of interest from the group of two-dimensional coronary artery angiogram images;
S300A, extracting a centerline of the blood vessel segment;
S400A, determining a difference in time taken for a contrast agent flowing through the blood vessel segment in any two frames of the two-dimensional coronary artery angiogram images with the difference being $\Delta t$, and determining a difference for the centerline of a sub-segment of the blood vessel segment in the two frames of two-dimensional coronary artery angiogram image with the difference being $\Delta L$;
$\Delta t = m \times fps$, since each group of two-dimensional coronary artery angiogram images contains multiple frames of two-dimensional coronary artery angiogram images played consecutively, m represents a difference in frame number between two frames of two-dimensional angiogram image selected from each group of two-dimensional coronary artery angiogram images, and *fps* represents an interval time for switching between two adjacent frames of the image, preferably, fps=1/15 second;
the blood flow velocity v is solved according to the ratio of $\Delta L$ to $\Delta t$.

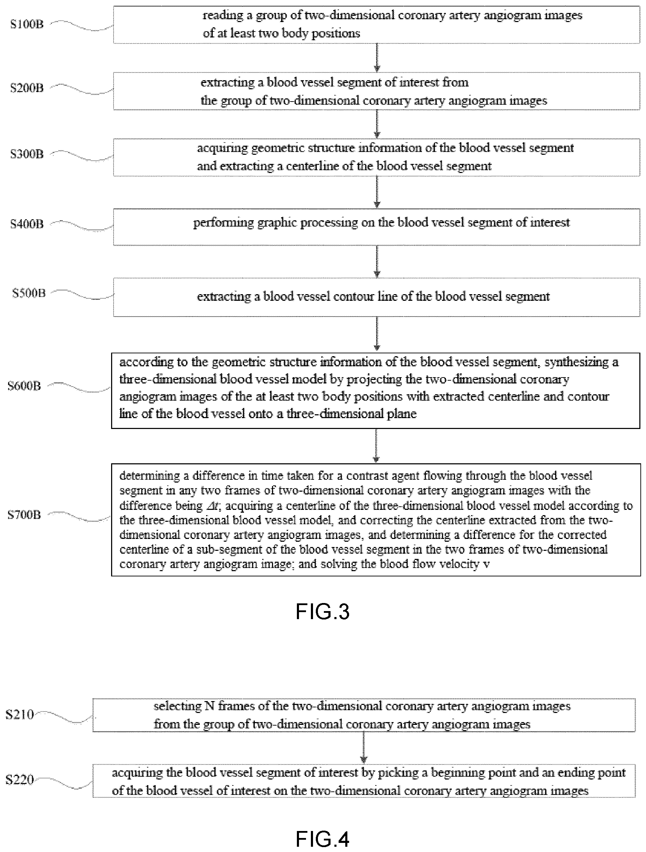

As shown in FIG. 3 , in an embodiment of the present disclosure, when acquiring the blood flow velocity by three-dimensional modeling, S010 comprises:

S100B, reading a group of two-dimensional coronary artery angiogram images of at least two body positions;
S200B, extracting a blood vessel segment of interest from the group of two-dimensional coronary artery angiogram images;
S300B, acquiring geometric structure information of the blood vessel segment and extracting a centerline of the blood vessel segment;
S400B, performing graphic processing on the blood vessel segment of interest;
S500B, extracting a blood vessel contour line of the blood vessel segment;
S600B, according to the geometric structure information of the blood vessel segment, synthesizing a three-dimensional blood vessel model by projecting the two-dimensional coronary angiogram images of the at least two body positions with extracted centerline and contour line of the blood vessel onto a three-dimensional plane;
S700B, determining a difference in time taken for a contrast agent flowing through the blood vessel segment in any two frames of two-dimensional coronary artery angiogram images with the difference being $\Delta t$; acquiring a centerline of the three-dimensional blood vessel model according to the three-dimensional blood vessel model, and correcting the centerline extracted from the two-dimensional coronary artery angiogram images, and determining a difference for the corrected centerline of a sub-segment of the blood vessel segment in the two frames of two-dimensional coronary artery angiogram image with the difference being $\Delta L'$; and solving the blood flow velocity v according to the ratio of $\Delta L'$ to $\Delta t$.

$\Delta t = m \times fps$, since each group of two-dimensional coronary artery angiogram images contains multiple frames of two-dimensional coronary artery angiogram images played consecutively, m represents a difference in frame number between two frames of two-dimensional angiogram image selected from each group of two-dimensional coronary artery angiogram images, and *fps* represents an interval time for switching between two adjacent frames of the image, preferably, *fps*=1/15 second.

In an embodiment of the present disclosure, S100A or S100B comprises:

directly reading the group of two-dimensional coronary artery angiogram images of at least one body position from an angiogram image capturing device or a hospital platform in a wireless or wired way; or reading the group of two-dimensional coronary artery angiogram images of at least one body position via a storage device.

As shown in FIG. 4 , in an embodiment of the present disclosure, S200A or S200B comprises:

S210, selecting N frames of the two-dimensional coronary artery angiogram images from the group of two-dimensional coronary artery angiogram images;
S220, acquiring the blood vessel segment of interest by picking a beginning point and an ending point of the blood vessel of interest on the two-dimensional coronary artery angiogram images.

Preferably, S200A or S200B further comprises: defining a first frame of the two-dimensional coronary artery angiogram image where a catheter appears as a reference image, and defining a k-th frame of the two-dimensional coronary artery angiogram image where a full coronary artery appears as a target image, wherein k is a positive integer greater than 1; subtracting the target image from the reference image to extract a feature point O of the catheter; preferably, removing a part of static noise; further, removing a part of dynamic noise by using a average filtering; and further denoising by means of gray histogram analysis and by using a threshold; extracting an image of the region where the coronary artery locates by subtracting the reference image from the target image; carrying out dynamic growth of the image of the region with the feature point of the catheter as a seed point to obtain an image of the blood vessel segment of interest.

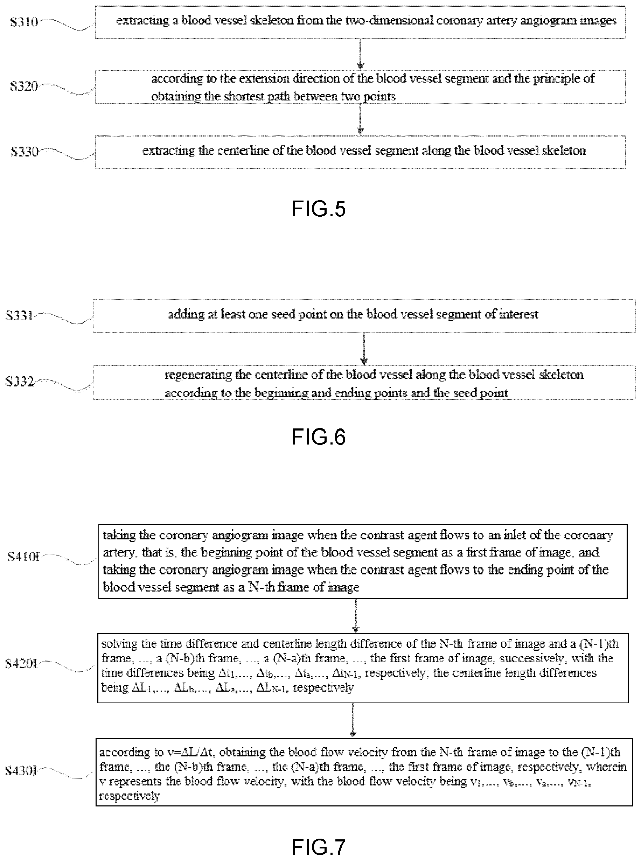

As shown in FIG. 5, in an embodiment of the present disclosure, S300A or S300B comprises:

S310, extracting a blood vessel skeleton from the two-dimensional coronary artery angiogram images;
S320, according to the extension direction of the blood vessel segment and the principle of obtaining the shortest path between two points;
S330, extracting the centerline of the blood vessel segment along the blood vessel skeleton.

As shown in FIG. 6, in an embodiment of the present disclosure, S330 further comprises:

S331, adding at least one seed point on the blood vessel segment of interest;
S332, regenerating the centerline of the blood vessel along the blood vessel skeleton according to the beginning and ending points and the seed point.

In an embodiment of the present disclosure, S400A or S700B comprises two acquisition methods. Method (1) is shown in FIG. 7, comprising:

S410I, taking the coronary angiogram image when the contrast agent flows to an inlet of the coronary artery, that is, the beginning point of the blood vessel segment as a first frame of image, and taking the coronary angiogram image when the contrast agent flows to the ending point of the blood vessel segment as a N-th frame of image;

S420I, solving the time difference and centerline length difference of the N-th frame of image and a (N-1)th frame, ..., a (N-b)th frame, ..., a (N-a)th frame, ..., the first frame of image, successively, with the time differences being $\Delta t_1,..., \Delta t_b,..., \Delta t_a,..., \Delta t_{N-1}$, respectively; the centerline length differences being $\Delta L_1,..., \Delta L_b,..., \Delta L_a,..., \Delta L_{N-1}$, respectively;

S430I, according to $v = \Delta L / \Delta t$, obtaining the blood flow velocity from the N-th frame of image to the (N-1)th frame, ..., the (N-b)th frame, ..., the (N-a)th frame, ..., the first frame of image, respectively, wherein v represents the blood flow velocity, with the blood flow velocity being $v_1,..., v_b,..., v_a,..., v_{N-1}$, respectively.

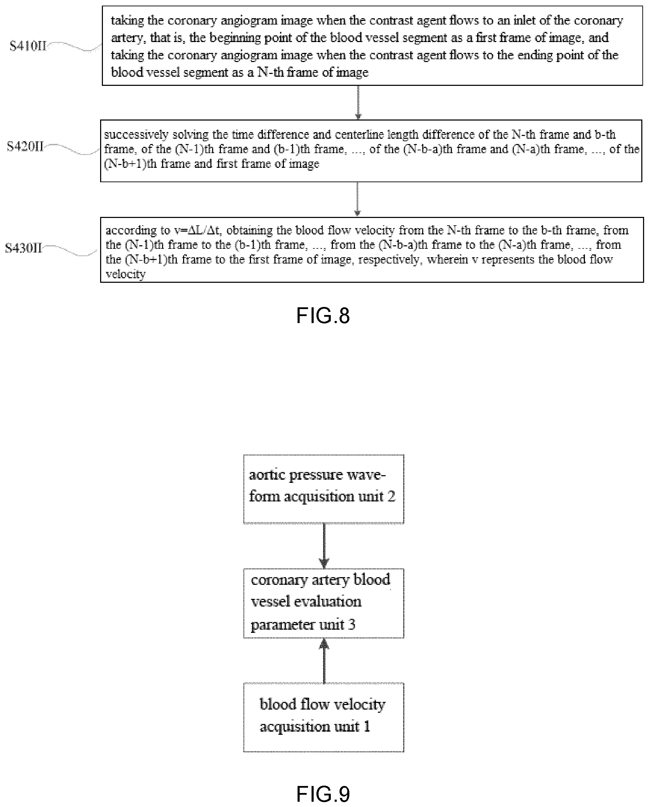

In an embodiment of the present disclosure, S400A or S700B comprises two acquisition methods. Method (2) is shown in FIG. 8 , comprising:

S410II, taking the coronary angiogram image when the contrast agent flows to an inlet of the coronary artery, that is, the beginning point of the blood vessel segment as a first frame of image, and taking the coronary angiogram image when the contrast agent flows to the ending point of the blood vessel segment as a N-th frame of image;

S420II, successively solving the time difference and centerline length difference of the N-th frame and b-th frame, of the (N-1)th frame and (b-1)th frame, ..., of the (N-b-a)th frame and (N-a)th frame, ..., of the (N-b+1)th frame and first frame of image.

S430II, according to $v = \Delta L / \Delta t$ , obtaining the blood flow velocity from the N-th frame to the b-th frame, from the (N-1)th frame to the (b-1)th frame, ..., from the (N-b-a)th frame to the (N-a)th frame, ..., from the (N-b+1)th frame to the first frame of image, respectively, wherein v represents the blood flow velocity.

In the present disclosure, recursive algorithm or bubbling algorithm may also be used to select a minimum value of the blood flow velocity, that is, the blood flow velocity during a systolic phase.

Embodiment 2:

As shown in FIG. 9, the present disclosure provides an apparatus for acquiring coronary artery blood vessel evaluation parameter based on physiological parameter, for use in the method for acquiring coronary artery blood vessel evaluation parameter based on physiological parameter according to any one of the above, comprising: a blood flow velocity acquisition unit 1, an aortic pressure waveform acquisition unit 2, a coronary artery blood vessel evaluation parameter unit 3. The coronary artery blood vessel evaluation parameter unit 3 is connected with the blood flow velocity acquisition unit 1 and the aortic pressure waveform acquisition unit 2. The blood flow velocity acquisition unit 1 is configured to obtain the blood flow velocity v. The aortic pressure waveform acquisition unit 2 is configured to acquire, in real time, an aortic pressure waveform changing over time. The coronary artery blood vessel evaluation parameter unit 3 is configured to receive the blood flow velocity v and the aortic pressure waveform sent by the blood flow velocity acquisition unit 1 and the aortic pressure waveform acquisition unit 2, and then to obtain a coronary artery evaluation parameter according to a physiological parameter.

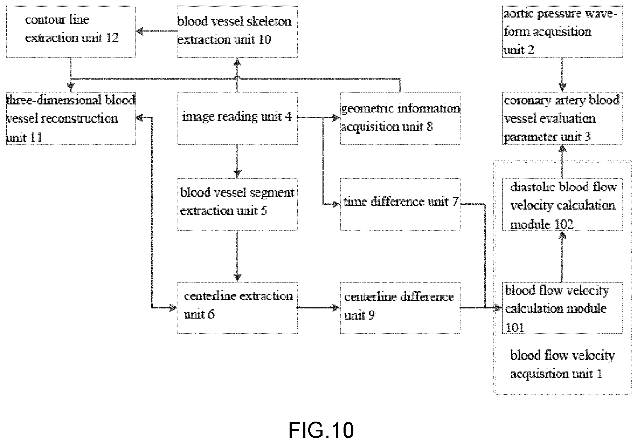

As shown in FIG. 10, in an embodiment of the present disclosure, the apparatus further comprises: an image reading unit 4, a blood vessel segment extraction unit 5, and a centerline extraction unit 6 connected in sequence, a time difference unit 7 and a geometric information acquisition unit 8 connected to the image reading unit 4, a centerline difference unit 9 connected with the centerline extraction unit 6. Both the time difference unit 7 and the centerline difference unit 9 are connected with the blood flow velocity acquisition unit 1. The geometric information acquisition unit 8 is connected with the coronary artery blood vessel evaluation parameter unit 3. The image reading unit 4 is configured to read a group of two-dimensional coronary artery angiogram images of at least one body position. The blood vessel segment extraction unit 5 is configured to receive two-dimensional coronary artery angiogram images sent by the image reading unit 4, and to extract a blood vessel segment of interest in the images. The centerline extraction unit 6 is configured to receive the blood vessel segment sent by the blood vessel segment extraction unit 5 and to extract the centerline of the blood vessel segment. The time difference unit 7 is configured to receive any two frames of the two-dimensional coronary artery angiogram images sent by the image reading unit 4 and to determining a difference in time taken for a contrast agent flowing through the blood vessel segment in the two frames of two-dimensional coronary artery angiogram image with the difference value being $\Delta t$. The centerline difference unit 9 is configured to receive the centerline of a sub-segment of the blood vessel segment flowed through by the contrast agent in the two frames of two-dimensional coronary artery angiogram image sent by the centerline extraction unit 6, and to determine a difference for the centerline of a sub-segment of the blood vessel segment in the two frames of two-dimensional coronary artery angiogram image with the difference being $\Delta L$. The blood flow velocity acquisition unit 1 comprises a blood flow velocity calculation module 101 and a diastolic blood flow velocity calculation module 102. The blood flow velocity calculation module 101 is connected with the time difference unit 7 and the centerline difference unit 8, respectively. The diastolic blood flow velocity calculation module 102 is connected with the blood flow velocity calculation module 101. The blood flow velocity calculation module 101 is configured to receive $\Delta L$ and the $\Delta t$ sent by the time difference unit 7 and the centerline difference unit 8, and to solve the blood flow velocity according to the ratio of $\Delta L$ to $\Delta t$. The diastolic blood flow velocity calculation module 102 is configured to receive the blood flow velocity value sent by the blood flow velocity calculation module, and to select a maximum value of the blood flow velocity as a blood flow velocity during a diastolic period. The geometric information acquisition unit 8 is configured to receive the two-dimensional coronary artery angiogram images of the image reading unit 4, to acquire a physiological parameter of a patient and image shooting angles, and to transmit the physiological parameter and the image shooting angles to the coronary artery blood vessel evaluation parameter unit 3.

In an embodiment of the present application, the apparatus further comprises: a blood vessel skeleton extraction unit 10 and a three-dimensional blood vessel reconstruction unit 11 both connected to the image reading unit 4, a contour line extraction unit 12 connected with the blood vessel skeleton extraction unit 10. The three-dimensional blood vessel reconstruction unit 11 is connected to the geometric information acquisition unit 8, the centerline extraction unit 6, and the contour line extraction unit 12. The blood vessel skeleton extraction unit 10 is configured to receive the two-dimensional coronary artery angiogram images sent by the image reading unit 4, and to extract the blood vessel skeleton in the images. The contour line extraction unit 12 is configured to receive a blood vessel skeleton of the blood vessel skeleton extraction unit 10, and to extract a contour line of the blood vessel segment of interest according to the blood vessel skeleton. The three-dimensional blood vessel reconstruction unit 11 is configured to receive the contour line, the geometric structure information and the centerline sent by the contour line extraction unit 12, the geometric information acquisition unit 8 and the centerline extraction unit 6, and to receive the two-dimensional coronary artery angiogram images sent by the image reading unit, which in order to synthesize a three-dimensional blood vessel model by projecting the two-dimensional coronary angiogram images of at least two body positions with extracted centerline and contour line of the blood vessel onto a three-dimensional plane and according to the geometric structure information of the blood vessel segment. The centerline extraction unit 6 is configured to re-extract the centerline of the blood vessel segment from the three-dimensional blood vessel model of the three-dimensional blood vessel reconstruction unit 11, and to re-acquire the length of the centerline.

The present disclosure provides a coronary artery analysis system, comprising: the apparatus for acquiring coronary artery blood vessel evaluation parameter based on physiological parameter according to any one of the above.

The present disclosure provides a computer storage medium, wherein the method for acquiring coronary artery blood vessel evaluation parameter based on physiological parameter according to any one of the above is implemented when a computer program is executed by a processor.

A person skilled in the art knows that various aspects of the present disclosure can be implemented as a system, a method, or a computer program product. Therefore, each aspect of the present disclosure can be specifically implemented in the following forms, namely: complete hardware implementation, complete software implementation (including firmware, resident software, microcode, etc.), or a combination of hardware and software implementations, which here can be collectively referred to as "circuit", "module" or "system". In addition, in some embodiments, various aspects of the present disclosure may also be implemented in the form of a computer program product in one or more computer-readable media, and the computer-readable medium contains computer-readable program code. Implementation of a method and/or a system of embodiments of the present disclosure may involve performing or completing selected tasks manually, automatically, or a combination thereof.

For example, hardware for performing selected tasks according to the embodiment(s) of the present disclosure may be implemented as a chip or a circuit. As software, selected tasks according to the embodiment(s) of the present disclosure can be implemented as a plurality of software instructions executed by a computer using any suitable operating system. In the exemplary embodiment(s) of the present disclosure, a data processor performs one or more tasks according to the exemplary embodiment(s) of a method and/or system as described herein, such as a computing platform for executing multiple instructions. Optionally, the data processor comprises a volatile memory for storing instructions and/or data, and/or a non-volatile memory for storing instructions and/or data, for example, a magnetic hard disk and/or movable medium. Optionally, a network connection is also provided. Optionally, a display and/or user input device, such as a keyboard or mouse, are/is also provided.

Any combination of one or more computer readable media can be utilized. The computer-readable medium may be a computer-readable signal medium or a computer-readable storage medium. The computer-readable storage medium may be, for example, but not limited to, an electrical, magnetic, optical, electromagnetic, infrared, or semiconductor system, apparatus, or device, or any combination of the above. More specific examples (non-exhaustive list) of computer-readable storage media would include the following:

Electrical connection with one or more wires, portable computer disk, hard disk, random access memory (RAM), read only memory (ROM), erasable programmable read only memory (EPROM or flash memory), optical fiber, portable compact disk read only memory (CD-ROM), optical storage device, magnetic storage device, or any suitable combination of the above. In this document, the computer-readable storage medium can be any tangible medium that contains or stores a program, and the program can be used by or in combination with an instruction execution system, apparatus, or device.

The computer-readable signal medium may include a data signal propagated in baseband or as a part of a carrier wave, which carries computer-readable program code. This data signal for propagation can take many forms, including but not limited to electromagnetic signals, optical signals, or any suitable combination of the above. The computer-readable signal medium may also be any computer-readable medium other than the computer-readable storage medium. The computer-readable medium can send, propagate, or transmit a program for use by or in combination with the instruction execution system, apparatus, or device.

The program code contained in the computer-readable medium can be transmitted by any suitable medium, including, but not limited to, wireless, wired, optical cable, RF, etc., or any suitable combination of the above.

For example, any combination of one or more programming languages can be used to write computer program codes for performing operations for various aspects of the present disclosure, including object-oriented programming languages such as Java, Smalltalk, C++, and conventional process programming languages, such as "C" programming language or similar programming language. The program code can be executed entirely on a user's computer, partly on a user's computer, executed as an independent software package, partly on a user's computer and partly on a remote computer, or entirely on a remote computer or server. In the case of a remote computer, the remote computer can be connected to a user's computer through any kind of network including a local area network (LAN) or a wide area network (WAN), or it can be connected to an external computer (for example, connected through Internet provided by an Internet service provider).

It should be understood that each block of the flowcharts and/or block diagrams and combinations of blocks in the flowcharts and/or block diagrams can be implemented by computer program instructions. These computer program instructions can be provided to the processor of general-purpose computers, special-purpose computers, or other programmable data processing devices to produce a machine, which produces a device that implements the functions/actions specified in one or more blocks in the flowcharts and/or block diagrams when these computer program instructions are executed by the processor of the computer or other programmable data processing devices.

It is also possible to store these computer program instructions in a computer-readable medium. These instructions make computers, other programmable data processing devices, or other devices work in a specific manner, so that the instructions stored in the computer-readable medium generate an article of manufacture comprising instructions for implementation of the functions/actions specified in one or more blocks in the flowcharts and/or block diagrams.

Computer program instructions can also be loaded onto a computer (for example, a coronary artery analysis system) or other programmable data processing equipment to facilitate a series of operation steps to be performed on the computer, other programmable data processing apparatus or other apparatus to produce a computer-implemented process, which enable instructions executed on a computer, other programmable device, or other apparatus to provide a process for implementing the functions/actions specified in the flowcharts and/or one or more block diagrams.

The above specific examples of the present disclosure further describe the purpose, technical solutions and beneficial effects of the present disclosure in detail. It should be understood that the above are only specific embodiments of the present disclosure and are not intended to limit the present disclosure. Within the spirit and principle of the present disclosure, any modification, equivalent replacement, improvement, etc. shall be included in the protection scope of the present disclosure.

**Claims**

1. A method for acquiring coronary artery blood vessel evaluation parameter based on physiological parameter, **characterized by** comprising:

   acquiring a physiological parameter;
   acquiring a blood flow velocity $v$;
   acquiring, in real time, an aortic pressure waveform changing over time;
   acquiring a coronary artery blood vessel evaluation parameter according to the blood flow velocity v, the aortic pressure waveform and the physiological parameter.

2. The method for acquiring coronary artery blood vessel evaluation parameter based on physiological parameter according to claim 1, **characterized in that** the coronary artery blood vessel evaluation parameter is an index for microcirculatory resistance of coronary artery CAIFMR during a diastolic phase, comprising:

　　selecting a maximum value of the blood flow velocity v, i.e., a maximum blood flow velocity $v_{max}$ during the diastolic phase;
　　a time period corresponding to the $v_{max}$ being the diastolic phase, acquiring an average aortic pressure during the diastolic phase according to the aortic pressure waveform;

$$CAIFMR = \overline{P_a}/v_{max} \times k + c;$$

$$\overline{P_a} = \frac{\sum_1^j \left(P_{a1} + P_{a2}\ldots P_{aj}\right)}{j};$$

　　wherein, $\overline{P_a}$ represents an average aortic pressure during the diastolic phase; $P_{a1}$, $P_{a2}$, and $P_{aj}$ represent aortic pressures corresponding to a first point, a second point, and a *j-th* point within the diastolic phase on the aortic pressure waveform, respectively, and *j* represents the number of pressure points contained in the aortic pressure waveform during the diastolic phase, $v_{max}$ represents a maximum blood flow velocity during the diastolic phase obtained by selecting a maximum value from all blood flow velocities v; k=axb, a represents a characteristic value of diabetes, and b represents a characteristic value of hypertension, and c represents gender.

3. The method for acquiring coronary artery blood vessel evaluation parameter based on physiological parameter according to claim 2, **characterized in that** if a patient does not suffer from diabetes, then 0.5≤a≤1; if the patient suffers from diabetes, then 1<a≤2;

　　if the patient's blood pressure is greater than or equal to 90 mmHg, then 1<b≤1.5; if the patient's blood pressure is less than 90 mmHg, then 0.5≤b≤1;
　　if the patient is male, then c=0; if the patient is female, then c=3~10.

4. The method for acquiring coronary artery blood vessel evaluation parameter based on physiological parameter according to claim 3, **characterized in that**, if the patient does not suffer from diabetes, then a=1; if the patient suffers from diabetes, then a=2;

　　if the patient's blood pressure is greater than or equal to 90mmHg, then b=1.5; if the patient's blood pressure is less than 90mmHg, then b=1; if the patient is male, c=0; if the patient is female, c=5.

5. The method for acquiring coronary artery blood vessel evaluation parameter based on physiological parameter according to claim 2, **characterized in that** a manner for acquiring a blood flow velocity comprises:

　　reading a group of two-dimensional coronary artery angiogram images of at least one body position;
　　extracting a blood vessel segment of interest from the group of two-dimensional coronary artery angiogram images;
　　extracting a centerline of the blood vessel segment;
　　determining a difference in time taken for a contrast agent flowing through the blood vessel segment in any two frames of the two-dimensional coronary artery angiogram images with the difference being $\Delta t$, and determining a difference for the centerline of a sub-segment of the blood vessel segment in the two frames of two-dimensional coronary artery angiogram image with the difference being $\Delta L$;
　　solving the blood flow velocity according to a ratio of $\Delta L$ to $\Delta t$.

6. The method for acquiring coronary artery blood vessel evaluation parameter based on physiological parameter according to claim 5, **characterized in that** a manner for extracting a blood vessel segment of interest from the group of two-dimensional coronary artery angiogram images comprises:

　　selecting N frames of the two-dimensional coronary artery angiogram images from the group of two-dimensional coronary artery angiogram images;
　　acquiring the blood vessel segment of interest by picking a beginning point and an ending point of the blood vessel of interest on the two-dimensional coronary artery angiogram images.

7. The method for measuring a blood flow velocity during the diastolic phase according to claim 5, **characterized in that** a manner for extracting the centerline of the blood vessel segment comprises:

　　extracting a blood vessel skeleton from the two-dimensional coronary artery angiogram images;
　　according to the extension direction of the blood vessel segment and the principle of obtaining the shortest path between two points;
　　extracting the centerline of the blood vessel segment along the blood vessel skeleton.

8. The method for acquiring coronary artery blood vessel evaluation parameter based on physiological parameter according to claim 6, **characterized in that** a manner for determining a difference in time taken for a contrast agent flowing through the blood vessel segment in any two frames of the two-dimensional coronary artery angiogram images with the difference being $\Delta t$, and determining a difference for the centerline of a sub-segment of the blood vessel segment in the two frames of two-dimensional coronary artery angiogram image with the difference being $\Delta L$, and solving the blood flow velocity according to the ratio of $\Delta L$ to $\Delta t$ comprises:

taking the coronary angiogram image when the contrast agent flows to the inlet of the coronary artery, that is, the beginning point of the blood vessel segment as a first frame of image, and taking the coronary angiogram image when the contrast agent flows to the ending point of the blood vessel segment as a N-th frame of image; solving the time difference and centerline length difference of the N-th frame of image and a (N-1)th frame, ..., a (N-b)th frame, ..., a (N-a)th frame, ..., the first frame of image, successively, with the time differences being $\Delta t_1$,..., $\Delta t_b$,..., $\Delta t_a$,..., $\Delta t_{N-1}$, respectively; the centerline length differences being $\Delta L_1$,..., $\Delta L_b$,..., $\Delta L_a$,..., $\Delta L_{N-1}$, respectively;

according to $v = \dfrac{\Delta L}{\Delta t}$ , obtaining the blood flow velocity from the N-th frame of image to the (N-1)th frame, ..., the (N-b)th frame, ..., the (N-a)th frame, ..., the first frame of image, respectively, wherein v represents the blood flow velocity, with the blood flow velocity being $v_1$,..., $v_b$,..., $v_a$,..., $v_{N-1}$, respectively.

9. The method for acquiring coronary artery blood vessel evaluation parameter based on physiological parameter according to claim 6, **characterized in that** a manner for determining a difference in time taken a contrast agent flowing through the blood vessel segment in any two frames of the two-dimensional coronary artery angiogram images with the difference being $\Delta t$, and determining a difference for the centerline of a sub-segment of the blood vessel segment in the two frames of two-dimensional coronary artery angiogram image with the difference being $\Delta L$, and solving the blood flow velocity according to the ratio of $\Delta L$ to $\Delta t$ comprises:

solving the time difference and centerline length difference of the N-th frame and b-th frame, of the (N-1)th frame and (b-1)th frame, ..., of the (N-b-a)th frame and (N-a)th frame, ..., of the (N-b+1)th frame and first frame of image, succes-

sively;

according to $v = \dfrac{\Delta L}{\Delta t}$ , obtaining the blood flow velocity from the N-th frame to the b-th frame, from the (N-1)th frame to the (b-1)th frame, ..., from the (N-b-a)th frame to the (N-a)th frame, ..., from the (N-b+1)th frame to the first frame of image, respectively, wherein v represents the blood flow velocity.

10. The method for acquiring coronary artery blood vessel evaluation parameter based on physiological parameter according to claim 5, **characterized in that**, after the manner for extracting a centerline of the blood vessel segment, and before the manner for determining a difference in time taken for a contrast agent flowing through the blood vessel segment in any two frames of the two-dimensional coronary artery angiogram images with the difference being $\Delta t$, and determining a difference for the centerline of a sub-segment of the blood vessel segment in the two frames of two-dimensional coronary artery angiogram image with the difference being $\Delta L$ ,further comprises:

reading a group of two-dimensional coronary artery angiogram images of at least two body positions; acquiring geometric structure information of the blood vessel segment; performing graphics processing on the blood vessel segment of interest; extracting a blood vessel contour line of the blood vessel segment; according to the geometric structure information of the blood vessel segment, synthesizing a three-dimensional blood vessel model by projecting the two-dimensional coronary angiogram images of the at least two body positions with extracted centerline and contour line of the blood vessel onto a three-dimensional plane.

11. The method for obtaining coronary artery evaluation parameter based on physiological parameter according to claim 10, **characterized in that** a manner for solving the blood flow velocity according to the ratio of $\Delta L$ to $\Delta t$ comprises:

according to the three-dimensional blood vessel model, acquiring a centerline of the three-dimensional blood vessel model, correcting the centerline extracted from the two-dimensional coronary angiogram images, and correcting the centerline difference $\Delta L$ to obtain $\Delta L'$; solving the blood flow velocity v according to the ratio of the $\Delta L'$ to the $\Delta t$.

**12.** An apparatus for acquiring coronary artery blood vessel evaluation parameter based on physiological parameter, for use in the method for acquiring coronary artery blood vessel evaluation parameter based on physiological parameter according to any one of claims 1 to 11, **characterized by** comprising: a blood flow velocity acquisition unit, an aortic pressure waveform acquisition unit, and a coronary artery blood vessel evaluation parameter unit, the coronary artery blood vessel evaluation parameter unit being connected to the blood flow velocity acquisition unit and the aortic pressure waveform acquisition unit;

the blood flow velocity obtaining unit being configured to obtain a blood flow velocity v;
the aortic pressure waveform acquisition unit being configured to acquire, in real time, an aortic pressure waveform changing over time;
the coronary artery blood vessel evaluation parameter unit being configured to receive the blood flow velocity v and the aortic pressure waveform sent by the blood flow velocity acquisition unit and the aortic pressure waveform acquisition unit, and then to obtain a coronary artery evaluation parameter according to a physiological parameter.

**13.** The apparatus for acquiring coronary artery blood vessel evaluation parameter based on physiological parameter according to claim 12, further comprising: an image reading unit, a blood vessel segment extraction unit, and a centerline extraction unit connected in sequence, a time difference unit and a geometric information acquisition unit connected to the image reading unit, the blood flow velocity acquisition unit being connected with the time difference unit and the centerline difference unit, respectively; the centerline difference unit being connected with the centerline extraction unit, the geometric information acquisition unit being connected with the coronary artery blood vessel evaluation parameter unit;

the image reading unit being configured to read a group of two-dimensional coronary artery angiogram image of at least one body position;
the blood vessel segment extraction unit being configured to receive two-dimensional coronary artery angiogram images sent by the image reading unit, and to extract a blood vessel segment of interest in the images;
the centerline extraction unit being configured to receive the blood vessel segment sent by the blood vessel segment extraction unit, and to extract the centerline of the blood vessel segment;
the time difference unit being configured to receive any two frames of the two-dimensional coronary artery angiogram images sent by the image reading unit, and to determine a difference in time taken for a contrast agent flowing through the blood vessel segment in the two frames of two-dimensional coronary artery angiogram image with the difference being $\Delta t$;
the centerline difference unit being configured to receive the centerline of a sub-segment of the blood vessel segment flowed through by the contrast agent in the two frames of two-dimensional coronary artery angiogram image sent by the centerline extraction unit, and to determine a difference for the centerline of a sub-segment of the blood vessel segment in the two frames of two-dimensional coronary artery angiogram image with the difference being $\Delta L$;
the blood flow velocity acquisition unit, comprising a blood flow velocity calculation module and a diastolic blood flow velocity calculation module, the blood flow velocity calculation module being respectively connected to the time difference unit and the centerline difference unit, the diastolic blood flow velocity calculation module being connected with the blood flow velocity calculation module;
the blood flow velocity calculation module being configured to receive the $\Delta L$ and the $\Delta t$ sent by the time difference unit and the centerline difference unit, and to solve the blood flow velocity according to the ratio of $\Delta L$ to $\Delta t$;
the diastolic blood flow velocity calculation module being configured to receive the blood flow velocity sent by the blood flow velocity calculation module, and to select a maximum value of the blood flow velocity as a blood flow velocity during a diastolic phase;
the geometric information acquisition unit being configured to receive the two-dimensional coronary artery angiogram images of the image reading unit, to acquire a physiological parameter of a patient and image shooting angles, and to transmit the physiological parameter and image shooting angles to the coronary artery blood vessel evaluation parameter unit.

**14.** The apparatus for acquiring coronary artery blood vessel evaluation parameter based on physiological parameter according to claim 13, further comprising: a blood vessel skeleton extraction unit and a three-dimensional blood vessel reconstruction unit, both connected to the image reading unit, a contour line extraction unit connected to the blood vessel skeleton extraction unit, the three-dimensional blood vessel reconstruction unit being connected with the geometric information acquisition unit, the centerline extraction unit and the contour line extraction unit;

the blood vessel skeleton extraction unit being configured to receive the two-dimensional coronary artery angiogram images sent by the image reading unit, and to extract a blood vessel skeleton in the images;

the contour line extraction unit being configured to receive the blood vessel skeleton of the blood vessel skeleton extraction unit, and to extract a contour line of the blood vessel segment of interest according to the blood vessel skeleton;

the three-dimensional blood vessel reconstruction unit being configured to receive the contour line, the image shooting angles and the centerline sent by the contour line extraction unit, the geometric information acquisition unit and the centerline extraction unit, and to receive the two-dimensional coronary artery angiogram images sent by the image reading unit in order to synthesize a three-dimensional blood vessel model by projecting the two-dimensional coronary angiogram images of at least two body positions with extracted centerline and contour line of the blood vessel onto a three-dimensional plane and according to the geometric structure information of the blood vessel segment;

the centerline extraction unit being configured to re-extract the centerline of the blood vessel segment from the three-dimensional blood vessel model of the three-dimensional blood vessel reconstruction unit, and to re-acquire the length of the centerline.

15. A coronary artery analysis system, **characterized by** comprising the apparatus for acquiring coronary artery blood vessel evaluation parameter based on physiological parameter according to any one of claims 12 to 14.

16. A computer storage medium, **characterized in that** the method for acquiring coronary artery blood vessel evaluation parameter based on physiological parameter according to any one of claims 1 to 11 is implemented when a computer program is executed by a processor.

S000 — acquiring a physiological parameter

S010 — acquiring a blood flow velocity v

S020 — acquiring, in real time, an aortic pressure waveform changing over time

S030 — acquiring the coronary artery blood vessel evaluation parameter according to the blood flow velocity v, the aortic pressure waveform and the physiological parameter

FIG.1

S100A — reading a group of two-dimensional coronary artery angiogram images of at least one body position

S200A — extracting a blood vessel segment of interest from the group of two-dimensional coronary artery angiogram images

S300A — extracting a centerline of the blood vessel segment

S400A — determining a difference in time taken for a contrast agent flowing through the blood vessel segment in any two frames of the two-dimensional coronary artery angiogram images with the difference being Δt, and determining a difference for the centerline of a sub-segment of the blood vessel segment in the two frames of two-dimensional coronary artery angiogram image with the difference being ΔL; solving the blood flow velocity v according to the ratio of ΔL to Δt

FIG.2

S100B — reading a group of two-dimensional coronary artery angiogram images of at least two body positions

S200B — extracting a blood vessel segment of interest from the group of two-dimensional coronary artery angiogram images

S300B — acquiring geometric structure information of the blood vessel segment and extracting a centerline of the blood vessel segment

S400B — performing graphic processing on the blood vessel segment of interest

S500B — extracting a blood vessel contour line of the blood vessel segment

S600B — according to the geometric structure information of the blood vessel segment, synthesizing a three-dimensional blood vessel model by projecting the two-dimensional coronary angiogram images of the at least two body positions with extracted centerline and contour line of the blood vessel onto a three-dimensional plane

S700B — determining a difference in time taken for a contrast agent flowing through the blood vessel segment in any two frames of two-dimensional coronary artery angiogram images with the difference being $\Delta t$; acquiring a centerline of the three-dimensional blood vessel model according to the three-dimensional blood vessel model, and correcting the centerline extracted from the two-dimensional coronary artery angiogram images, and determining a difference for the corrected centerline of a sub-segment of the blood vessel segment in the two frames of two-dimensional coronary artery angiogram image; and solving the blood flow velocity v

FIG.3

S210 — selecting N frames of the two-dimensional coronary artery angiogram images from the group of two-dimensional coronary artery angiogram images

S220 — acquiring the blood vessel segment of interest by picking a beginning point and an ending point of the blood vessel of interest on the two-dimensional coronary artery angiogram images

FIG.4

| S310 | extracting a blood vessel skeleton from the two-dimensional coronary artery angiogram images |
| S320 | according to the extension direction of the blood vessel segment and the principle of obtaining the shortest path between two points |
| S330 | extracting the centerline of the blood vessel segment along the blood vessel skeleton |

## FIG.5

| S331 | adding at least one seed point on the blood vessel segment of interest |
| S332 | regenerating the centerline of the blood vessel along the blood vessel skeleton according to the beginning and ending points and the seed point |

## FIG.6

| S410I | taking the coronary angiogram image when the contrast agent flows to an inlet of the coronary artery, that is, the beginning point of the blood vessel segment as a first frame of image, and taking the coronary angiogram image when the contrast agent flows to the ending point of the blood vessel segment as a N-th frame of image |
| S420I | solving the time difference and centerline length difference of the N-th frame of image and a (N-1)th frame, ..., a (N-b)th frame, ..., a (N-a)th frame, ..., the first frame of image, successively, with the time differences being $\Delta t_1,..., \Delta t_b,..., \Delta t_a,..., \Delta t_{N-1}$, respectively; the centerline length differences being $\Delta L_1,..., \Delta L_b,..., \Delta L_a,..., \Delta L_{N-1}$, respectively |
| S430I | according to $v=\Delta L/\Delta t$, obtaining the blood flow velocity from the N-th frame of image to the (N-1)th frame, ..., the (N-b)th frame, ..., the (N-a)th frame, ..., the first frame of image, respectively, wherein v represents the blood flow velocity, with the blood flow velocity being $v_1,..., v_b,..., v_a,..., v_{N-1}$, respectively |

## FIG.7

| S410II | taking the coronary angiogram image when the contrast agent flows to an inlet of the coronary artery, that is, the beginning point of the blood vessel segment as a first frame of image, and taking the coronary angiogram image when the contrast agent flows to the ending point of the blood vessel segment as a N-th frame of image |
|---|---|

| S420II | successively solving the time difference and centerline length difference of the N-th frame and b-th frame, of the (N-1)th frame and (b-1)th frame, ..., of the (N-b-a)th frame and (N-a)th frame, ..., of the (N-b+1)th frame and first frame of image |
|---|---|

| S430II | according to v=ΔL/Δt, obtaining the blood flow velocity from the N-th frame to the b-th frame, from the (N-1)th frame to the (b-1)th frame, ..., from the (N-b-a)th frame to the (N-a)th frame, ..., from the (N-b+1)th frame to the first frame of image, respectively, wherein v represents the blood flow velocity |
|---|---|

FIG.8

aortic pressure wave-form acquisition unit 2

coronary artery blood vessel evaluation parameter unit 3

blood flow velocity acquisition unit 1

FIG.9

| contour line extraction unit 12 | ← | blood vessel skeleton extraction unit 10 | | aortic pressure wave-form acquisition unit 2 |

| three-dimensional blood vessel reconstruction unit 11 | image reading unit 4 | geometric information acquisition unit 8 | coronary artery blood vessel evaluation parameter unit 3 |

| blood vessel segment extraction unit 5 | time difference unit 7 | diastolic blood flow velocity calculation module 102 |

| centerline extraction unit 6 | centerline difference unit 9 | blood flow velocity calculation module 101 |

blood flow velocity acquisition unit 1

FIG.10

## INTERNATIONAL SEARCH REPORT

| International application No. |
| --- |
| **PCT/CN2019/116664** |

**A.  CLASSIFICATION OF SUBJECT MATTER**

A61B 5/026(2006.01)i

According to International Patent Classification (IPC) or to both national classification and IPC

**B.  FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNPAT, CNKI, WPI, EPODOC: 生理, 参数, 数据, 微循环阻力, 评定, 评价, 分数, 男, 女, 性别, 糖尿病, 血流, 速度, 速率, 动脉, 血管, 冠状动脉, IFMR, CAIFMR, IMR, physiology, parameter, data, microcirculation, resistance, assessment, evaluation, score, male, female, gender, diabetes, blood, flow, speed, velocity, artery, vessel, coronary

**C.  DOCUMENTS CONSIDERED TO BE RELEVANT**

| Category* | Citation of document, with indication, where appropriate, of the relevant passages | Relevant to claim No. |
| --- | --- | --- |
| Y | CN 108742587 A (BODONG MEDICAL IMAGING TECHNOLOGY (SHANGHAI) CO., LTD.) 06 November 2018 (2018-11-06)<br>    description paragraphs [0003]-[0004], [0055]-[0104], [0126]-[0133], [0174] | 1, 12-16 |
| Y | CN 108550189 A (SUZHOU RAINMED MEDICAL TECHNOLOGY CO., LTD.) 18 September 2018 (2018-09-18)<br>    description, paragraphs [0071]-[0121] | 1, 12-16 |
| A | CN 109065170 A (BODONG MEDICAL IMAGING TECHNOLOGY (SHANGHAI) CO., LTD.) 21 December 2018 (2018-12-21)<br>    entire document | 1-16 |
| A | CN 109009037 A (BODONG MEDICAL IMAGING TECHNOLOGY (SHANGHAI) CO., LTD.) 18 December 2018 (2018-12-18)<br>    entire document | 1-16 |
| A | CN 108735270 A (HANGZHOU ARTERYFLOW TECHNOLOGY CO., LTD.) 02 November 2018 (2018-11-02)<br>    entire document | 1-16 |

☑ Further documents are listed in the continuation of Box C.　　☑ See patent family annex.

| | |
| --- | --- |
| *     Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| **07 July 2020** | **11 August 2020** |

| Name and mailing address of the ISA/CN | Authorized officer |
| --- | --- |
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2019/116664** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 106456026 A (ST JUDE MEDICAL SYSTEMS AB) 22 February 2017 (2017-02-22)<br>entire document | 1-16 |
| A | 徐亮 等 (XU, Liang et al.). "血糖控制水平对糖尿病患者冠状动脉微循环阻力指数的影响 (Effect of blood glucose control level on coronary index of microcirculatory resistance in diabetic patients)"<br>东南大学学报(医学版) (Journal of Southeast University(Medical Science Edition) ),<br>Vol. 37, No. 2,, 30 April 2018 (2018-04-30),<br>pp. 315-318 | 1-16 |

Form PCT/ISA/210 (second sheet) (January 2015)

INTERNATIONAL SEARCH REPORT
Information on patent family members

International application No.

**PCT/CN2019/116664**

| Patent document cited in search report | | | Publication date (day/month/year) | Patent family member(s) | | | Publication date (day/month/year) |
|---|---|---|---|---|---|---|---|
| CN | 108742587 | A | 06 November 2018 | WO | 2019242161 | A1 | 26 December 2019 |
| CN | 108550189 | A | 18 September 2018 | WO | 2019210553 | A1 | 07 November 2019 |
| CN | 109065170 | A | 21 December 2018 | WO | 2019242159 | A1 | 26 December 2019 |
| CN | 109009037 | A | 18 December 2018 | | None | | |
| CN | 108735270 | A | 02 November 2018 | | None | | |
| CN | 106456026 | A | 22 February 2017 | US | 2015313478 | A1 | 05 November 2015 |
| | | | | JP | 2019193786 | A | 07 November 2019 |
| | | | | CA | 2944114 | A1 | 08 October 2015 |
| | | | | EP | 3125751 | A2 | 08 February 2017 |
| | | | | AU | 2015242353 | A1 | 29 September 2016 |
| | | | | US | 2019365247 | A1 | 05 December 2019 |
| | | | | WO | 2015150913 | A2 | 08 October 2015 |
| | | | | JP | 2017510412 | A | 13 April 2017 |

Form PCT/ISA/210 (patent family annex) (January 2015)